# EUROPEAN PATENT APPLICATION

(11) **EP 3 418 392 A1**
(43) Date of publication of application: **26.12.2018**
(21) Application number: 17753325.4
(22) Date of filing: 17.02.2017
(51) Int. Cl.: C12Q 1/68, C12N 15/09, G01N 33/53, G01N 33/574

(54) **CANCER CELL DETERMINATION METHOD USING FOXB2 GENE**

(30) Priority: 17.02.2016 JP 2016028394
(71) Applicant: Fujifilm Wako Pure Chemical Corporation, Osaka-shi, Osaka 540-8605 (JP)
(72) Inventor: HAYASHIDA, Yukinobu, Amagasaki-shi, Hyogo 661-0963 (JP)
(74) Representative: Winter, Brandl, Fürniss, Hübner, Röss, Kaiser, Polte - Partnerschaft mbB
(86) International application number: PCT/JP2017/005947
(87) International publication number: WO 2017/142070

(57) **Abstract**

An object of the present invention is to provide a method that enables easy determination of cancer cells.

The present invention relates to "a cancer cell determination method including the following steps 1 to 3: (1) step 1 of subjecting a cell-derived nucleic acid to a bisulfite reaction, the nucleic acid containing at least a base sequence represented by SEQ ID NO: 2 and containing part or all of a base sequence represented by SEQ ID NO: 1; (2) step 2 of amplifying, among reaction products obtained in step 1, a bisulfite reaction product of a nucleic acid containing at least the base sequence represented by SEQ ID NO: 2 and consisting of part or all of the base sequence represented by SEQ ID NO: 1; and (3) step 3 of determining whether or not the cell is a cancer cell on the basis of the results of subjecting the amplified product obtained in step 2 to electrophoresis", "a primer set including a forward primer consisting of a base sequence represented by SEQ ID NO: 6 and a reverse primer consisting of a base sequence containing part or all of SEQ ID NO: 8 and containing at least a base sequence represented by SEQ ID NO: 7 or a reverse primer consisting of a base sequence represented by SEQ ID NO: 9" and "a cell cancer determination reagent including the primer set".

SEQUENCE LISTING

<110>  FUJIFILM WAKO PURE CHEMICAL CORPORATION

<120>  CANCER CELL DETERMINATION METHOD USING FOXB2 GENE

<130>  WP0160P-WOEP

<140> EP17753325.4
<141> 2018-08-16

<150>  JP2016028394
<151>  2016-02-17

<160>  26

<170>  PatentIn version 3.5

<210>  1
<211>  500
<212>  DNA
<213>  Human FOXB2

<400>  1
ggtctggcta agtgcctggc caggggcctg cgtccagggc actggaagtc ctgctgtcac      60

tggcctccag gacagcaggc gcaaactcag atttaaaggg ccagagtctc tactgcccag     120

accttcctcc cagccatctt ggtctgggtt tccctcggac ctagaaaaag caaagaggcc     180

gggaggaaac acggcccctt gccactctcc tctgcagcca gctccagcac tcagtctttg     240

gccacccggg ggaagacgca gagaaggcgg cggtaaacct ggtgcactcc gcccgcgact     300

gtgcgcgcca gtcggcaacc gtcggggcca gaagttgcca gcttccgaga gctgagtagg     360

cccgaaaggc caaggtcgga gacaccaggc aattcggaga aggcaggaga gagaagcaga     420

gagggcctgg agggcgagag ggcaaagtgg cgggactgga ggggccgagt gggaattagt     480

gggggcagcc aggccccagg                                                 500

<210>  2
<211>  150
<212>  DNA
<213>  Human FOXB2

<400>  2
cgggggaaga cgcagagaag gcggcggtaa acctggtgca ctccgcccgc gactgtgcgc      60

gccagtcggc aaccgtcggg gccagaagtt gccagcttcc gagagctgag taggcccgaa     120

aggccaaggt cggagacacc aggcaattcg                                      150

<210>  3
<211>  212
<212>  DNA
<213>  Human FOXB2

<400>  3
gcagccagct ccagcactca gtctttggcc acccggggga agacgcagag aaggcggcgg      60

taaacctggt gcactccgcc cgcgactgtg cgcgccagtc ggcaaccgtc ggggccagaa 120

gttgccagct tccgagagct gagtaggccc gaaaggccaa ggtcggagac accaggcaat 180

tcggagaagg caggagagag aagcagagag gg 212

<210> 4
<211> 217
<212> DNA
<213> Human FOXB2

<400> 4
gcagccagct ccagcactca gtctttggcc acccggggga agacgcagag aaggcggcgg 60

taaacctggt gcactccgcc cgcgactgtg cgcgccagtc ggcaaccgtc ggggccagaa 120

gttgccagct tccgagagct gagtaggccc gaaaggccaa ggtcggagac accaggcaat 180

tcggagaagg caggagagag aagcagagag ggcctgg 217

<210> 5
<211> 287
<212> DNA
<213> Human FOXB2

<400> 5
gcagccagct ccagcactca gtctttggcc acccggggga agacgcagag aaggcggcgg 60

taaacctggt gcactccgcc cgcgactgtg cgcgccagtc ggcaaccgtc ggggccagaa 120

gttgccagct tccgagagct gagtaggccc gaaaggccaa ggtcggagac accaggcaat 180

tcggagaagg caggagagag aagcagagag ggcctggagg gcgagagggc aaagtggcgg 240

gactggaggg gccgagtggg aattagtggg ggcagccagg ccccagg 287

<210> 6
<211> 28
<212> DNA
<213> Artificial

<220>
<223> Primer

<400> 6
gtagttagtt ttagtattta gtttttgg 28

<210> 7
<211> 29
<212> DNA
<213> Artificial

<220>
<223> Primer

<400> 7
ccctctctac ttctctctcc taccttctc 29

<210>  8
<211>  34
<212>  DNA
<213>  Artificial

<220>
<223>  Primer

<400>  8
ccaaaccctc tctacttctc tctcctacct tctc                                    34


<210>  9
<211>  29
<212>  DNA
<213>  Artificial

<220>
<223>  Primer

<400>  9
ccttaatcac ccccatcaat ccaaaatcc                                          29


<210>  10
<211>  41
<212>  DNA
<213>  Artificial

<220>
<223>  Primer

<400>  10
ttaccataag cttgtagtta gttttagtat ttagtttttg g                            41


<210>  11
<211>  42
<212>  DNA
<213>  Artificial

<220>
<223>  Primer

<400>  11
taattaagga tccccctctc tacttctctc tcctaccttc tc                           42


<210>  12
<211>  28
<212>  DNA
<213>  Artificial

<220>
<223>  Primer

<400>  12
gtagaggaaa tagaatattt tagttaag                                           28


<210>  13
<211>  28

<212>  DNA
<213>  Artificial

<220>
<223>  Primer

<400>  13
cttaaccaat aaaaatcact tccaactc                                          28


<210>  14
<211>  227
<212>  DNA
<213>  Human FOXB1

<400>  14
gcagaggaaa cagaataccc cagccaagca atttccatgt caaacatcat ccgcgcggct      60

gctccatctg tgaacgtgag tggtcgcctg gctccctctt ccgcggcggc cgcttcctca     120

taccttcaca cggcgcacac cggccggcgc gtccagctgc ctgcccagtg gccgaggctc     180

ttccccctcg cccagtcttg agctggaagt gattcctatt ggccaag                   227


<210>  15
<211>  29
<212>  DNA
<213>  Artificial

<220>
<223>  Primer

<400>  15
ggaattagtg ggggtagtta ggttttagg                                         29


<210>  16
<211>  28
<212>  DNA
<213>  Artificial

<220>
<223>  Primer

<400>  16
cccaaaaact acccttacca aactaatc                                          28


<210>  17
<211>  24
<212>  DNA
<213>  Artificial

<220>
<223>  Primer

<400>  17
ggtaaatttg gtgtattttg tttg                                              24


<210>  18
<211>  24

<212>  DNA
<213>  Artificial

<220>
<223>  Primer

<400>  18
ggtaaatttg gtgtatttcg ttcg                                              24


<210>  19
<211>  24
<212>  DNA
<213>  Artificial

<220>
<223>  Primer

<400>  19
caaaaactaa caacttctaa cccc                                              24


<210>  20
<211>  24
<212>  DNA
<213>  Artificial

<220>
<223>  Primer

<400>  20
cgaaaactaa caacttctaa cccc                                              24


<210>  21
<211>  26
<212>  DNA
<213>  Artificial

<220>
<223>  Primer

<400>  21
gttgttagtt tttgagagtt gagtag                                            26


<210>  22
<211>  26
<212>  DNA
<213>  Artificial

<220>
<223>  Primer

<400>  22
gttgttagtt ttcgagagtt gagtag                                            26


<210>  23
<211>  29
<212>  DNA
<213>  Artificial

<220>
<223>  Primer

<400>  23
ccctctctac ttctctctcc taccttctc                                        29


<210>  24
<211>  355
<212>  DNA
<213>  Human FOXB2

<400>  24
ggaattagtg ggggcagcca ggccccagga acggagtgcg gagagattct gtggtccagt      60

gcgggccgga gggcggtgga ggagccgggg gcgatgccgc ggccggggaa gagctcgtac     120

agcgaccaaa aaccgcccta ctcttacatc tcgctgaccg ccatggcaat ccagcactcg     180

gccgagaaga tgctgccgct gagcgacatc tacaagttca tcatggagcg cttcccctac     240

taccgcgagc acacacagcg ctggcagaac agcctgcgcc acaacctctc cttcaacgac     300

tgcttcatca agattccgcg gaggcccgac cagcctggca agggtagctt ctggg          355


<210>  25
<211>  76
<212>  DNA
<213>  Humen FOXB2

<400>  25
ggtaaacctg gtgcactccg cccgcgactg tgcgcgccag tcggcaaccg tcggggccag      60

aagttgccag cttccg                                                      76


<210>  26
<211>  92
<212>  DNA
<213>  Human FOXB2

<400>  26
gttgccagct tccgagagct gagtaggccc gaaaggccaa ggtcggagac accaggcaat      60

tcggagaagg caggagagag aagcagagag gg                                    92

## Description

### Technical Field

The present invention relates to a cancer cell determination method using a FOXB2 gene.

### Background Art

A group of transcription factors with a forkhead or winged-helix DNA binding domain is called forkhead box (FOX), and there are about 50 types thereof in humans. The FOX primarily functions as a developmental regulatory factor, a tissue-specific regulatory factor, or a cell cycle regulatory factor, but there are many types whose actions are little elucidated. Among them, FOXB2 has been reported to be involved in embryonic neurogenesis in *Xenopus laevis,* but its function in mammals has not yet been elucidated.

Meanwhile, methylation of DNA has been known to be associated with cancerization, and JP2008-283947A (Patent Literature 1) discloses a method for detecting esophageal cancer on the basis of a methylation rate in a CpG island (CpG dinucleotide: -CG- sequence) of a FOXB2 gene.

### Citation List

### Patent Literature

[Patent Literature 1] JP2008-283947A

### Summary of Invention

### Technical Problem

Patent Literature 1 discloses that cancer cells often undergo methylation of cytosine of a CpG dinucleotide and also discloses that cytosine of a CpG dinucleotide was confirmed to be methylated by measuring the FOXB2 gene of esophageal cancer by a BAMCA method. However, in order to determine whether or not a cell is a cancer cell by this method, it was necessary to decode the base sequence and then check whether or not cytosine of a CpG dinucleotide was methylated. Therefore, a method of easily determining whether or not a cell is a cancer cell has been desired. That is, it is an object of the present invention to provide a method enabling easy determination of a cancer cell.

### Solution to Problem

As a result of extensive research on a method for detecting a bisulfite reaction product as a method of easily distinguishing between cancer cells and normal cells, the present inventors have found that amplified products derived from cancer cells and amplified products derived from normal cells can be separated by amplification and electrophoresis of a nucleic acid containing a specific base sequence in a promoter region of a FOXB2 gene after being subjected to a bisulfite reaction. Since the base sequence to be amplified after being subjected to the bisulfite reaction has the same length whether it is a cancer cell or a normal cell, it was considered difficult to separate amplified products thereof by electrophoresis. However, it was unexpectedly found that amplified products derived from cancer cells and amplified products derived from normal cells can be separated in the case of amplifying a nucleic acid containing a specific base sequence. The present inventors have completed the present invention on the basis of these findings.

That is, the present invention relates to "a cancer cell determination method including the following steps 1 to 3: (1) step 1 of subjecting a cell-derived nucleic acid to a bisulfite reaction, the nucleic acid containing(comprising) at least a base sequence represented by SEQ ID NO: 2 and containing part or all of a base sequence represented by SEQ ID NO: 1; (2) step 2 of amplifying, among reaction products obtained in step 1, a bisulfite reaction product of a nucleic acid containing at least the base sequence represented by SEQ ID NO: 2 and consisting of part or all of the base sequence represented by SEQ ID NO: 1; and (3) step 3 of determining whether or not the cell is a cancer cell on the basis of the results of subjecting the amplified product obtained in step 2 to electrophoresis", "a primer set including a forward primer consisting of a base sequence represented by SEQ ID NO: 6 and a reverse primer consisting of a base sequence containing part or all of SEQ ID NO: 8 and containing at least a base sequence represented by SEQ ID NO: 7 or a reverse primer consisting of a base sequence represented by SEQ ID NO: 9", and "a cell cancer determination reagent including the primer set".

### Advantageous Effects of Invention

According to the method of the present invention, it can be determined whether or not a target cell is a cancer cell by amplifying a nucleic acid containing a specific base sequence of a bisulfite reaction product, and then subjecting the resulting amplified product to electrophoresis, with no need to decode a base sequence, unlike a conventional method, and without carrying out a restriction enzyme treatment or the like. That is, it can be determined whether or not the target cell is a cancer cell in a short time with a very simple method. In addition, since it is not necessary to decode a base sequence and to carry out a restriction enzyme treatment or the like, there is also an advantage that cost and labor required for the base sequence decoding and restriction enzyme treatment can be reduced. In addition, according to the method of the present invention, a benign tumor can be determined as a non-cancer cell, and therefore the determination of cancer can be made with high accuracy.

### (Brief Description of Drawings)

Fig. 1 shows the results of electrophoresis of a PCR amplified product by the method of the present invention using normal cells of hiPS and cancer cells of human glioma cells (U251-MG-P1) in Example 1.
Fig. 2 shows the results of analysis of cytosine methylation using normal cells of hiPS and cancer cells of U251-MG-P1 in Experimental Example 1.
Fig. 3 shows the results of electrophoresis of a PCR amplified product obtained using a plasmid of a FOXB2 promoter region of normal cells of hiPS and cancer cells of U251-MG-P1 in Example 2, and the results of electrophoresis of a PCR amplified product of a FOXB1 gene using normal cells of hiPS and cancer cells of human glioma cells (U251-MG-P1) in Comparative Example 1.
Fig. 4 shows the results of electrophoresis of amplified sequence 1 of a FOXB2 gene using normal cells of hiPS and cancer cells of U251-MG-P1 in Comparative Example 2.
Fig. 5 shows the results of electrophoresis of amplified sequence 2 of a FOXB2 gene using normal cells of hiPS and cancer cells of U251-MG-P1 in Comparative Example 2.
Fig. 6 shows the results of electrophoresis of amplified sequence 3 of a FOXB2 gene using normal cells of hiPS and cancer cells of U251-MG-P1 in Comparative Example 2.
Fig. 7-1 shows the results of electrophoresis of a PCR amplified product of a FOXB2 gene using cells derived from breast cancer patients (normal tissue, tumor, and lymph node) in Example 3.
Fig. 7-2 shows the results of electrophoresis of a PCR amplified product of a FOXB2 gene using cells derived from breast cancer patients (normal tissue, tumor, and lymph node) in Example 3.
Fig. 7-3 shows the results of electrophoresis of a PCR amplified product of a FOXB2 gene using cells derived from breast cancer patients (normal tissue, tumor, and lymph node) in Example 3.
Fig. 8-1 shows the results of electrophoresis of a PCR amplified product of a FOXB2 gene using cells derived from colorectal cancer patients (normal tissue and tumor) in Example 4.
Fig. 8-2 shows the results of electrophoresis of a PCR amplified product of a FOXB2 gene using cells derived from colorectal cancer patients (normal tissue and tumor) in Example 4.

### Description of Embodiments

### Cancer cell determination method of present invention

The cancer cell determination method of the present invention (hereinafter, sometimes abbreviated as the method of the present invention) includes (1) step 1 of subjecting a cell-derived nucleic acid to a bisulfite reaction, the nucleic acid containing at least a base sequence represented by SEQ ID NO: 2 and containing part or all of a base sequence represented by SEQ ID NO: 1; (2) step 2 of amplifying, among reaction products obtained in step 1, a bisulfite reaction product of a nucleic acid containing at least the base sequence represented by SEQ ID NO: 2 and consisting of part or all of the base sequence represented by SEQ ID NO: 1; and (3) step 3 of determining whether or not the cell is a cancer cell on the basis of the results of subjecting the amplified product obtained in step 2 to electrophoresis.

### Step 1

The nucleic acid in step 1 is a nucleic acid which contains at least a base sequence represented by SEQ ID NO: 2 and contains part or all of a base sequence represented by SEQ ID NO: 1 and may be derived from a cell.

The type of cell is selected depending on the cancer to be determined, and examples thereof include cells derived from epithelial cells of a kidney, a uterus, a liver, a mammary gland, an ovary, a large intestine (a colon and a rectum), a prostate, a lung, a pancreas, and the like; leukocytes such as neutrophils, eosinophils, basophils, lymphocytes, and monocytes; cells derived from hematopoietic organs, such as bone marrow cells and T cells; cells derived from non-epithelial cells, such as a sarcoma; and cells derived from nerves, such as neuroblasts, in which cells derived from epithelial cells are preferable, and among them, cells derived from epithelial cells of a mammary gland, a lung, a large intestine, and the like are preferable. In addition, the cell is preferably derived from a mammal, and particularly preferably derived from a human.

In addition, the cells can be obtained by a method known per se, from samples, for example, biological samples derived from tissues, cells and body fluids such as blood (whole blood, serum, and plasma), lymph fluid, urine, nipple discharge, and feces, and cultures thereof.

The nucleic acid in step 1 may be extracted from the cells, and the extraction method may be carried out on the basis of a DNA extraction method known per se. Specific examples of the extraction method of DNA include a method of extracting DNA by mixing a sample with a treatment liquid containing a surfactant (sodium cholate, sodium dodecyl sulfate, or the like), and subjecting the resulting mixed solution to a physical treatment (agitation, homogenization, ultrasonic grinding, or the like) to thereby liberate the DNA contained in the sample into the mixed solution, a method of extracting DNA from a sample using phenol/chloroform, and a column extraction method. Among them, a method of extracting DNA from a sample using phenol/chloroform and a column extraction method are preferable.

The method of extracting DNA from a sample using phenol/chloroform is a method of extracting DNA excluding proteins present in the sample using phenol which is a protein denaturing agent and chloroform which promotes denaturation of proteins. A specific method thereof may be carried out on the basis of a method known per se, or may be carried out using a commercially available kit [for example, Phase Lock Gel (manufactured by QuantaBio)].

In addition, the column extraction method is a method in which a sample is added to a cylindrical container such as a column having a membrane or the like therein, and the DNA is extracted from the sample, utilizing a difference in size, adsorption power, charge, hydrophobicity, or the like of substances. A specific method thereof may be carried out on the basis of a method known per se and may be carried out using a commercially available kit [for example, QuickGene SP kit DNA tissue (manufactured by Kurabo Industries Ltd.)].

As for the nucleic acid in step 1, for example, in the case of determining liver cancer, a nucleic acid which is a hepatocyte-derived nucleic acid, contains at least the base sequence represented by SEQ ID NO: 2, and contains part or all of the base sequence represented by SEQ ID NO: 1 may be used; and, for example, in the case of determining prostate cancer, a nucleic acid which is a prostate cell-derived nucleic acid, contains at least the base sequence represented by SEQ ID NO: 2, and contains part or all of the base sequence represented by SEQ ID NO: 1 may be used. In addition, in the case where it is determined whether or not the leukocyte is a cancer cell, a nucleic acid which is a leukocyte-derived nucleic acid, contains at least the base sequence represented by SEQ ID NO: 2, and contains part or all of the base sequence represented by SEQ ID NO: 1 may be used using leukocytes taken out (separated) from the blood or the blood itself.

The nucleic acid in step 1 may be a nucleic acid containing at least the base sequence represented by SEQ ID NO: 2 present in a promoter region of a FOXB2 gene and containing part or all of the base sequence represented by SEQ ID NO: 1. The base sequence represented by SEQ ID NO: 1 is a part of the FOXB2 gene and is a base sequence of a region presumed to be a promoter region of the FOXB2 gene. It should be noted that, in the present specification, the FOXB2 gene contains the structural gene of the FOXB2 protein as well as its promoter region, enhancer region, silencer region, and the like.

Preferred examples of the nucleic acid in step 1 include genomic DNA, a nucleic acid containing at least the base sequence represented by SEQ ID NO: 3 and consisting of part or all of the base sequence represented by SEQ ID NO: 1, a nucleic acid containing at least the base sequence represented by SEQ ID NO: 4 and consisting of part or all of the base sequence represented by SEQ ID NO: 1, and a nucleic acid containing at least the base sequence represented by SEQ ID NO: 5 and consisting of part or all of the base sequence represented by SEQ ID NO: 1, among which genomic DNA is particularly preferable.

It should be noted that the base sequence represented by SEQ ID NO: 1 has been described as bases 106566 to 107065 in Genbank Accession No. AL 353637; the base sequence represented by SEQ ID NO: 2 has been described as bases 106812 to 106961 in Genbank Accession No. AL 353637; the base sequence represented by SEQ ID NO: 3 has been described as bases 106779 to 106990 in Genbank Accession No. AL 353637; the base sequence represented by SEQ ID NO: 4 has been described as bases 106779 to 106995 in Genbank Accession No. AL 353637; and the base sequence represented by SEQ ID NO: 5 has been described as bases 106779-107065 in Genbank Accession No. AL 353637.

Step 1 may be carried out in accordance with a per se known bisulfite method described, for example, in WO2013/089063. Specifically, for example, step 1 is carried out in such a manner that the nucleic acid extracted from the cell is subjected to a single strand formation treatment as necessary, and is reacted with sulfite to sulfonate cytosine, and the sulfonated cytosine is hydrolyzed, followed by desulfonation in the presence of an alkali. According to this reaction, methylated cytosine does not react and remains intact, and only unmethylated cytosine is converted into uracil.

In step 1, the bisulfite reaction may be carried out in the presence of at least one of the compounds represented by the following general formulae [1] to [8] described in WO2013/089063. Specific examples of the substituents (R₁ to R₃₉) in these compounds, specific examples of the compounds, amounts of the compounds to be used, methods of use thereof, and the like in that case may be set and carried out according to the description in WO2013/089063.

Compound represented by the general formula [1] 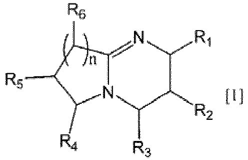 (In the formula, R₁ to R₆ each independently represent hydrogen or an alkyl group having 1 to 6 carbon atoms, and n represents an integer of 1 to 3.)

Compound represented by the general formula [2]

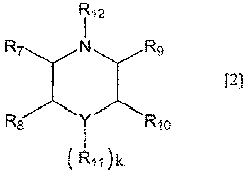

(In the formula, Y represents a carbon atom, an oxygen atom, or a nitrogen atom, R₇ to R₁₂ each independently represent a hydrogen atom or an alkyl group having 1 to 6 carbon atoms, and k represents an integer of 0 to 2, provided that k represents 0 in the case where Y is an oxygen atom, k represents 1 in the case where Y is a nitrogen atom, and k represents 2 in the case where Y is a carbon atom.)

Compound represented by the general formula [3]

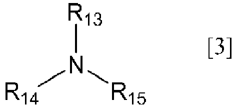

(In the formula, R₁₃ to R₁₅ each independently represent a hydrogen atom or an alkyl group having 1 to 6 carbon atoms.)

Compound represented by the general formula [4]

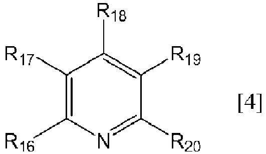

(In the formula, R₁₇ and R₁₉ each independently represent a hydrogen atom, an amino group, or an alkyl group having 1 to 6 carbon atoms, R₁₆, R₁₈, and R₂₀ each independently represent a hydrogen atom, an amino group, an alkyl group having 1 to 6 carbon atoms, or a dialkylamino group having 2 to 6 carbon atoms, and R₁₆ and R₁₇ may form a benzene ring together with a carbon atom adjacent to R₁₆ and a carbon atom adjacent to R₁₇.)

Compound represented by the general formula [5]

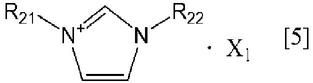

(In the formula, R₂₁ and R₂₂ each independently represent an alkyl group having 1 to 6 carbon atoms, and X₁ represents a counter anion.)

Compound represented by the general formula [6]

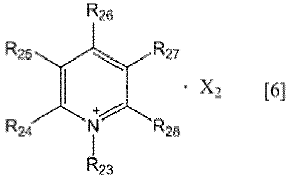

(In the formula, R₂₃ represents an alkyl group having 1 to 6 carbon atoms, R₂₄ to R₂₈ each independently represent a hydrogen atom or an alkyl group having 1 to 6 carbon atoms, and X₂ represents a counter anion.)

Compound represented by the general formula [7]

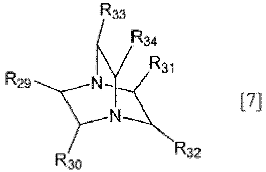

(In the formula, R₂₉ to R₃₄ each independently represent a hydrogen atom or an alkyl group having 1 to 6 carbon atoms.)

Compound represented by the general formula [8]

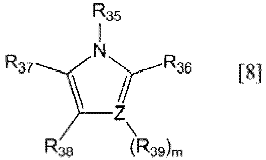

[In the formula, Z represents a nitrogen atom or a carbon atom, and m represents an integer of 0 or 1, provided that m represents 0 in the case where Z is a nitrogen atom, and m represents 1 in the case where Z is a carbon atom. R₃₅ to R₃₉ each independently represent hydrogen or an alkyl group having 1 to 6 carbon atoms.]

The nucleic acid in step 1 is used in a state in which the nucleic acid is dissolved in a solution such as water (sterile water) or a buffer solution. Examples of the buffer solution include a Good's buffer solution such as MES or HEPES, a phosphate buffer solution, a Tris buffer solution, a glycine buffer solution, a borate buffer solution, and a sodium hydrogen carbonate buffer solution, each of which has a pH of 6 to 8. As the solution used herein, sterile water is preferable. The amount of DNA in the solution is not particularly limited, but it is usually 10 ng to 1 µg in 1 to 10 µL of the solution.

The single strand formation treatment of the nucleic acid in step 1 may be carried out by a single strand formation treatment known per se. The single strand formation treatment may be carried out, for example, by a heat treatment involving heating usually at 80°C to 100°C, preferably 85°C to 95°C, usually for 30 seconds to 10 minutes, preferably 1 to 3 minutes, or may be carried out, for example, by an alkali treatment involving bringing the nucleic acid in step 1 into contact with an alkali and the like, but a heat treatment is preferred. In particular, in the case where the bisulfite reaction is carried out in the presence of the compound represented by any one of the general formulae [1] to [8], since the reaction can proceed without decomposition of the DNA even in the case where the reaction temperature is set to 80°C to 100°C, the nucleic acid in step 1 may be subjected to a bisulfite reaction at 80°C to 100°C and the single strand formation treatment and the bisulfite reaction may proceed at the same time. The treatment is simplified, so that the method is more preferable in the case of single strand formation treatment.

Specifically, for example, the alkali treatment is carried out by adding an alkali or an aqueous solution thereof to the nucleic acid in step 1 or a solution containing the nucleic acid to render the solution alkaline, usually pH 10 to 14, preferably 12 to 14. Examples of the alkali include alkali metal hydroxides such as sodium hydroxide and potassium hydroxide, alkaline earth metal hydroxides such as barium hydroxide, magnesium hydroxide, and calcium hydroxide, alkali metal carbonates such as sodium carbonate, ammonia, and amines. Among them, alkali metal hydroxides such as sodium hydroxide and potassium hydroxide are preferable, and sodium hydroxide is particularly preferable among them.

In the bisulfite reaction, examples of the sulfite in the reaction of nucleic acid with sulfite in step 1 include sodium hydrogen sulfite and ammonium sulfite, among which sodium hydrogen sulfite is preferable. The amount of sulfite used is added so that the final concentration in the reaction solution is usually 1 to 6 mol/L with respect to 1 to 500 µL of the solution containing 50 to 500 ng of nucleic acid in step 1. The reaction of nucleic acid with sulfite in step 1 is carried out usually at 30°C to 100°C, preferably 50°C to 100°C, more preferably 80°C to 95°C, usually for 60 minutes to 20 hours, preferably 60 minutes to 5 hours, more preferably 60 minutes to 3 hours. By this reaction, cytosine is sulfonated.

The method of hydrolysis of sulfonated cytosine in the bisulfite reaction is not particularly limited as long as it is usually done in the art, but it is carried out by heating usually at 30°C to 100°C, preferably 80°C to 95°C, usually for 60 minutes to 20 hours, preferably for 60 minutes to 5 hours, and more preferably for 60 minutes to 3 hours. It should be noted that the hydrolysis treatment may proceed simultaneously with the reaction of nucleic acid with sulfite in step 1. In that case, the reaction temperature and the reaction time in the reaction of nucleic acid with sulfite in step 1 may be set according to the hydrolysis conditions of the sulfonated cytosine.

The nucleic acid in step 1 subjected to the hydrolysis is preferably subjected to a purification treatment prior to a desulfonation treatment. The purification treatment is carried out for the purpose of removing a high concentration of sulfite or the like used in the bisulfite reaction and may be carried out in accordance with a method of purifying DNA commonly carried out in the art. Specific examples of the purification method include a method of adding a chaotropic agent such as guanidine hydrochloride, sodium iodide, or the like to the nucleic acid in step 1 or a solution containing the nucleic acid in step 1, followed by separation and purification by a HPLC method or the like, a method of extraction and purification with a phenol/chloroform/isoamyl alcohol mixed solution, an alcohol precipitation method, a method of purification with a column packed with silica gel, and a filter filtration method. Among them, an alcohol precipitation method is preferable. The alcohol precipitation method is specifically as follows.

That is, usually, 40 to 110 µL of alcohol and 30 to 100 µL of a buffer solution are added to 10 µL of the solution containing nucleic acid in step 1 after the hydrolysis treatment and centrifugation is carried out. After centrifugation, the supernatant is removed, followed by washing with alcohol, whereby the nucleic acid in step 1 can be separated and purified. At the time of adding the alcohol and the buffer solution, 0.1 to 1 µL of ethachinmate or glycogen may be added to 10 µL of the solution containing the nucleic acid in step 1 in order to facilitate removal of the supernatant after separation. Examples of the alcohol include ethanol, isopropanol, and butanol, among which isopropanol is particularly preferable. In the case where isopropanol is used, only DNA can be efficiently precipitated, and therefore the reaction can proceed efficiently. Examples of the buffer solution include a Good's buffer solution such as MES or HEPES; a phosphate buffer solution; a Tris buffer solution; a glycine buffer solution; a borate buffer solution; and a sodium hydrogen carbonate buffer solution, among which a Good's buffer solution such as MES or HEPES, a Tris buffer solution, or the like is preferable, and a Tris buffer solution is particularly preferable. The pH of these buffer solutions is usually 7 to 8 and preferably 7 to 7.5, and the concentration of a buffering agent in the buffer solution is usually in a range of 0.1 to 5 mol/L and preferably 0.1 to 2 mol/L. The centrifugation is not particularly limited as long as it is an aspect commonly carried out in the art, but is usually carried out under 12,000 to 22,000 g for 10 to 30 minutes.

The desulfonation reaction in the presence of an alkali in the bisulfite reaction includes the same method as alkali treatment in the section of the single strand formation treatment, and a preferred aspect thereof also includes the same one. Specifically, for example, the desulfonation reaction may be carried out as follows.

That is, the desulfonation reaction is carried out by adding usually 1 to 10 µL, preferably 1 to 5 µL of a 0.5 to 3 mol/L aqueous alkaline solution to 10 µL of the solution after hydrolysis treatment or the solution subjected to purification treatment after hydrolysis treatment, adjusting the pH of the reaction solution to 11 to 14, and preferably 12 to 14, and warming the solution usually at 25°C to 70°C, preferably at 30°C to 50°C, usually for 5 to 60 minutes, preferably for 5 to 30 minutes.

Preferred specific examples of step 1 (bisulfite reaction) will be described below.

That is, for example, genomic DNA is extracted from a target cell containing genomic DNA by a method known per se using a DNA extraction kit or the like, and then 1 µg of genomic DNA is dissolved in, for example, 10 to 30 µL of sterile water. To 3 to 5 µL of the solution, for example, 50 to 100 µL of 2 to 5 mol/L sodium hydrogen sulfite (pH: 5.0 to 7.0) and 1 to 12 µL of the compounds represented by the general formulae [1] to [8] or an aqueous solution containing those compounds is added, so that the concentration of the compound in a reaction solution is 3% to 10% in the case where the compounds represented by the general formulae [1] to [8] are liquids, and the concentration of the compound in the reaction solution is 1 to 1,000 mmol/L in the case where the compounds represented by the general formulae [1] to [8] are solids, and then the reaction solution is heated at 80°C to 100°C for 60 minutes to 3 hours. By the reaction, the genomic DNA becomes single-stranded DNA, cytosine in the single-stranded DNA is sulfonated, and simultaneously, sulfonated cytosine can be hydrolyzed. Subsequently, a 1 mol/L Tris buffer solution (pH: 7.0 to 8.0) and isopropanol are added each in 5 to 10 times amount of the solution after hydrolysis, in a ratio of 40:60 to 60:40, and preferably 40:60 to 50:50, to precipitate the DNA after hydrolysis. In this case, confirmation of DNA precipitation becomes easy in the case where 1 to 3 µL of ethachinmate or glycogen is added. Then, centrifugation is carried out under 12,000 to 20,000 g for 10 to 20 minutes to remove the supernatant, and the resulting DNA is washed with ethanol. By this treatment, DNA after hydrolysis can be extracted and purified. In addition, 1 µg of the resulting DNA is dissolved in, for example, 30 to 40 µL of sterile water, and 5 to 20 µL of 1 to 3 mol/L sodium hydroxide is added to the solution which is then subjected to a reaction at 30°C to 40°C for 20 to 60 minutes to carry out desulfonation of the DNA. Then, if necessary, the purification is carried out to remove low molecular weight DNA by using, for example, a commercially available kit or the like. By this treatment, the bisulfite reaction according to the present invention is completed, and DNA in which unmethylated cytosine in the genomic DNA is efficiently converted into uracil (bisulfite-treated nucleic acid (bisulfite reaction product)) can be obtained.

According to step 1, a nucleic acid in which nucleic acid in step 1 has been subjected to a bisulfite reaction, that is, a bisulfite reaction product can be obtained.

More specifically, unmethylated cytosine in the nucleic acid in step 1 is converted into uracil (U) by subjecting the nucleic acid in step 1 to a bisulfite reaction. On the other hand, methylated cytosine in the nucleic acid in step 1 remains cytosine (C) without the base being converted.

That is, the bisulfite reaction product has a sequence in which unmethylated cytosine in the nucleic acid in step 1 is substituted with uracil.

Therefore, the bisulfite reaction product can also be referred to as a "nucleic acid corresponding to the nucleic acid in step 1" on the basis of the nucleic acid in step 1 subjected to the bisulfite reaction in step 1.

### Step 2

Step 2 is a step of amplifying the bisulfite reaction product of a nucleic acid containing at least the base sequence represented by SEQ ID NO: 2 and consisting ofpart or all of the base sequence represented by SEQ ID NO: 1, among the reaction products obtained in step 1.

The nucleic acid to be amplified in step 2 (hereinafter, sometimes abbreviated as a target nucleic acid according to the present invention) is a nucleic acid (bisulfite reaction product), which is obtained by subjecting nucleic acid containing at least the base sequence represented by SEQ ID NO: 2 and consisting of part or all of the base sequence represented by SEQ ID NO: 1 to the bisulfite raction, among the nucleic acids (bisulfite reaction products) after the bisulfite reaction in step 1.

In other words, the target nucleic acid according to the present invention is a nucleic acid whose base sequence before being subjected to the bisulfite reaction in step 1 contains the base sequence represented by each SEQ ID NO. and is all or a part of the bisulfite reaction product (bisulfite reaction product of the nucleic acid) obtained by subjecting the nucleic acid to the bisulfite reaction.

As the target nucleic acid according to the present invention, for example, preferred is a bisulfite reaction product of a nucleic acid containing at least the base sequence represented by SEQ ID NO: 2 and consisting of part or all of the base sequence represented by SEQ ID NO: 5 (a nucleic acid in which a nucleic acid containing at least the base sequence represented by SEQ ID NO: 2 and consisting of part or all of the base sequence represented by SEQ ID NO: 5 has been subjected to the bisulfite reaction); more preferred is a bisulfite reaction product of a nucleic acid containing at least the base sequence represented by SEQ ID NO: 2 and consisting of part or all of the base sequence represented by SEQ ID NO: 4 (a nucleic acid in which a nucleic acid containing at least the base sequence represented by SEQ ID NO: 2 and consisting of part or all of the base sequence represented by SEQ ID NO: 4 has been subjected to the bisulfite reaction); and particularly preferred is a bisulfite reaction product of a nucleic acid containing at least the base sequence represented by SEQ ID NO: 2 and consisting of part or all of the base sequence represented by SEQ ID NO: 3 (a nucleic acid in which a nucleic acid containing at least the base sequence represented by SEQ ID NO: 2 and consisting of part or all of the base sequence represented by SEQ ID NO: 3 has been subjected to the bisulfite reaction).

In addition, the target nucleic acid according to the present invention is a nucleic acid having the base sequence represented by SEQ ID NO: 2 and a base sequence contiguous to its 3' end or/and 5' end.

The number of bases in the target nucleic acid according to the present invention is usually 150 to 510, preferably 150 to 290, more preferably 150 to 220, and still more preferably 150 to 212.

More specifically, unmethylated cytosine in a nucleic acid is converted into U (uracil) by subjecting the nucleic acid to a bisulfite reaction. On the other hand, methylated cytosine in the nucleic acid remains C (cytosine) without the base being converted.

For example, in the case where the nucleic acid in step 1 is [····CG····CG····] and all of the Cs therein are methylated, the base sequence amplified in step 2 (nucleic acid corresponding to the nucleic acid of step 1) is a sequence of [····CG····CG····] itself. In addition, in the case where the nucleic acid of step 1 is [····CG····CG····] and all of the Cs therein are not methylated, the base sequence amplified in step 2 (nucleic acid corresponding to the nucleic acid of step 1) is a sequence of [····UG····UG····]. In addition, in the case where the nucleic acid of step 1 is [····CG····CG····], a part of Cs therein is methylated (Cs on the left), and a part of Cs is not methylated (Cs on the right), the base sequence amplified in step 2 (nucleic acid corresponding to the nucleic acid of step 1) is a sequence of [····CG····UG····].

Thus, the base sequence of the nucleic acid amplified in step 2 is changed depending on the presence or absence of methylated cytosine in the nucleic acid (nucleic acid before being subjected to a bisulfite reaction) in step 1 and the position of methylated cytosine.

Therefore, the target nucleic acid according to the present invention contains the case where it has various base sequences as described above, and it may be, for example, a nucleic acid corresponding to a nucleic acid containing at least the base sequence represented by SEQ ID NO: 2 and consisting of part or all of the base sequence represented by SEQ ID NO: 1; preferably a nucleic acid corresponding to a nucleic acid containing at least the base sequence represented by SEQ ID NO: 2 and consisting of part or all of the base sequence represented by SEQ ID NO: 5; more preferably a nucleic acid corresponding to a nucleic acid containing at least the base sequence represented by SEQ ID NO: 2 and consisting of part or all of the base sequence represented by SEQ ID NO: 5; and still more preferably a nucleic acid corresponding to a nucleic acid containing at least the base sequence represented by SEQ ID NO: 2 and consisting of part or all of the base sequence represented by SEQ ID NO: 3.

As a method of amplifying the target nucleic acid according to the present invention, the bisulfite reaction product may be subjected to a PCR reaction using a primer designed to amplify the target nucleic acid according to the present invention.

The PCR reaction may be carried out according to a method known per se, for example, the method described in Nucleic Acids Research, 1991, Vol. 19, 3749, and Bio Techniques, 1994, Vol. 16, 1134-1137, and is specifically carried out as follows. That is, the target nucleic acid according to the present invention can be amplified by adding, to 1 to 100 ng of nucleic acid amount of the bisulfite reaction product as a template, two types of primers each usually in 0.1 to 100 pmol, and preferably 0.1 to 50 pmol, DNA polymerase usually in 0.05 to 10 units, preferably 0.1 to 5 units, and four types of mixed deoxyribonucleotide triphosphates (dNTPs) usually in 0.1 to 10 nmol and preferably 1 to 10 nmol, and by carrying out, for example, a reaction at 93°C to 98°C for 1 to 10 minutes, then a reaction of 20 to 40 cycles, each cycle including reaction (1) at 92°C to 98°C for 10 to 30 seconds → (2) at 50°C to 60°C for 10 to 30 seconds → (3) at 68°C to 74°C for 30 seconds to 5 minutes, and a further reaction at 68°C to 74°C for 1 to 10 minutes, in a buffer solution such as a tricine buffer solution or a Tris-HCl buffer solution, having a pH of 7 to 9.

In the PCR reaction, it is preferred that the resulting DNA is purified after the reaction, by a purification method commonly carried out in the art, for example, by a method for extraction with a mixed solution of phenol/chloroform/isoamyl alcohol, alcohol precipitation, column purification, filter filtration, or the like. In addition, it is more preferred that DNA having the target base pair (bp) is extracted after the purification. Examples of the extraction method include methods known per se, for example, a method using agarose gel electrophoresis, a method using liquid chromatography, and a method using electrophoresis on polyacrylamide gel described in an enlarged edition of Laboratory Manual for Genetic Engineering, 1990, 27-28. In addition, DNA obtained by the PCR reaction (PCR product) may be further subjected to the PCR reaction under the same conditions to yield more quantity.

The two types of primers in the PCR reaction may be those designed to amplify the target nucleic acid according to the present invention. Specifically, the forward primer may be designed to anneal to the upstream side of the base sequence represented by SEQ ID NO: 2 and the reverse primer may be designed to anneal to the downstream side of the base sequence represented by SEQ ID NO: 2. The number of nucleotides thereof is usually 20 to 45, preferably 22 to 40, and more preferably 25 to 35. Specific examples of the forward primer include those consisting of the sequence as set forth in SEQ ID NO: 6 below. The reverse primer may be, for example, a primer including a contiguous base sequence consisting of part or all of SEQ ID NO: 8 and containing at least the base sequence represented by SEQ ID NO: 7, or a primer consisting of the base sequence represented by SEQ ID NO: 9; preferably a primer including a contiguous base sequence consisting of part or all of SEQ ID NO: 8 and containing at least the base sequence represented by SEQ ID NO: 7; and more preferably a primer consisting of the base sequence represented by SEQ ID NO: 7.

Forward primer
   GTAGTTAGTT TTAGTATTTA GTTTTTGG (SEQ ID NO: 6)
Reverse primer
   CCCTCTCTA CTTCTCTCTC CTACCTTCTC (SEQ ID NO: 7)
   CCAAACCCTC TCTACTTCTC TCTCCTACCT TCTC (SEQ ID NO: 8)
   CCTTAATCAC CCCCATCAAT CCAAAATCC (SEQ ID NO: 9)

In step 2 of the method of the present invention, it is preferable to use the forward primer or/and the reverse primer as it is, and the primer may be labeled with a labeling substance to be a labeled primer. As the labeling substance, any known labeling substance such as a radioisotope, an enzyme, a fluorescent substance, a luminescent substance, or biotin can be used. Specific examples of the radioisotope include ³²P, ³³P, and ³⁵S. Specific examples of the enzyme include alkaline phosphatase and horseradish peroxidase. Specific examples of the fluorescent substance include Cyanine Dyes Cy3 and Cy5 (GE Healthcare), and fluorescein. Specific examples of the luminescent substance include chemiluminescent reagents containing acridinium ester.

As a method of labeling the primer with a radioisotope, there are a method in which a primer is labeled by incorporating a nucleotide labeled with a radioisotope in the case of synthesizing a primer; a method in which a primer is synthesized and then labeled with a radioisotope; and the like. Specific examples thereof include a random primer method, a nick translation method, a 5'-terminal labeling method using a T4 polynucleotide kinase, a 3'-terminal labeling method using a terminal deoxynucleotide transferase or the like, and an RNA labeling method, which are commonly used.

The method of labeling the primer with an enzyme may be, for example, a direct labeling method which is a conventional method in the art, such as directly covalently binding an enzyme molecule such as alkaline phosphatase or horseradish peroxidase to a primer for labeling.

As a method of labeling the primer with a fluorescent substance, for example, a fluorescein-labeled nucleotide may be incorporated into a primer by a conventional labeling method in the art. In addition, nucleotides can also be labeled with a fluorescent substance by a method of replacing a nucleotide having a linker arm as a member of oligonucleotides of a sequence (see, for example, Nucleic Acids Res., 1986, Vol. 14, p. 6115). In that case, there is a method in which uridine having a linker arm at the 5-position is chemically synthesized from deoxyuridine by the synthesis method disclosed in JP1985-500717A (JP-S60-500717A) and a fluorescent substance is introduced into the oligonucleotide chain.

Labeling of the primer with a luminescent substance or biotin can be carried out according to a conventional method in which a nucleotide is labeled with a luminescent label or biotin, which is commonly carried out in the art.

It should be noted that, in the PCR reaction, the sequence from the sequence to which the forward primer anneals to the sequence to which the reverse primer anneals (a region from the complementary sequence of the forward primer to the complementary sequence of the reverse primer containing the complementary sequence of SEQ ID NO: 2) is a base sequence (nucleic acid) to be amplified.

The DNA polymerase in the PCR reaction may be any DNA polymerase as long as it is commonly used in the art and DNA polymerase having a 5'→3' polymerase activity is preferable, among which DNA polymerase having an exonuclease activity but having no 3'→-5' exonuclease activity is more preferable. Specifically, for example, a mutant type or mixed type Taq DNA polymerase such as KAPA2G polymerase or Blend Taq-plus, Taq DNA polymerase, or Tth DNA polymerase is preferable, among which KAPA2G polymerase is particularly preferable.

The dNTPs in the PCR reaction are not particularly limited as long as they are a mixture of four types of deoxyribonucleotide triphosphates (dATP, dCTP, dGTP, and dTTP), commonly used in the art.

Preferred specific examples of step 2 (amplification method of target nucleic acid according to the present invention) will be described below.

That is, first, a nucleic acid extracted from the cells is subjected to a single strand formation treatment as described in the section of [Bisulfite reaction], and then subjected to a bisulfite reaction to obtain a bisulfite reaction product. Thereafter, the resulting bisulfite reaction product is subjected to a PCR reaction using primers designed to amplify the target nucleic acid according to the present invention. Specifically, 0.5 to 2 µL of 1 to 20 µmol/L forward primer (for example, SEQ ID NO: 6) of DNA to be amplified, 0.5 to 2 µL of 1 to 20 µmol/L reverse primer (for example, SEQ ID NO: 7, SEQ ID NO: 8, or SEQ ID NO: 9) of DNA to be amplified, 1 to 10 µL of a mixed solution of four types of 1 to 5 mmol/L deoxyribonucleotide triphosphates (dNTPs), and 0.1 to 10 µL of 0.1 to 5 units of Blend Taq-plus DNA polymerase are added to 0.5 to 2 µL of a solution containing 1 to 100 ng of the bisulfite reaction product obtained by the bisulfite reaction, and the reaction is carried out under conditions of, for example, a reaction at 92°C to 98°C for 1 to 5 minutes → a reaction of 20 to 40 cycles, each cycle including reaction [at 93°C to 98°C for 10 to 30 seconds → at 50°C to 60°C for 10 to 30 seconds → at 68°C to 74°C for 30 seconds to 5 minutes] → a reaction at 68°C to 74°C for 1 to 10 min. In this way, the target nucleic acid according to the present invention, in which unmethylated cytosine has been converted into uracil, is amplified. Specifically, in the case of a combination of the forward primer of SEQ ID NO: 6 and the reverse primer of SEQ ID NO: 7, the nucleic acid containing the base sequence represented by SEQ ID NO: 3 is amplified. In addition, in the case of a combination of the forward primer of SEQ ID NO: 6 and the reverse primer of SEQ ID NO: 8, the nucleic acid containing the base sequence represented by SEQ ID NO: 4 is amplified, and in the case of a combination of the forward primer of SEQ ID NO: 6 and the reverse primer of SEQ ID NO: 9, the nucleic acid containing the base sequence represented by SEQ ID NO: 5 is amplified.

### Step 3

The electrophoresis in step 3 may be on the basis of a method of migrating a substance at a different speed or migrating a substance by a different distance depending on the charge intensity of the substance. Specifically, for example, isoelectric focusing or gel electrophoresis such as SDS-polyacrylamide gel electrophoresis, agarose gel electrophoresis, or acrylamide gel electrophoresis can be used. Among them, preferred is electrophoresis utilizing a molecular sieving effect that a substance having a larger molecular weight migrates slower or a migration distance thereof becomes shorter, and more preferred is electrophoresis which allows separation of molecules on the basis of the difference in three-dimensional structures of the molecule. Specifically, gel electrophoresis such as SDS-polyacrylamide gel electrophoresis, agarose gel electrophoresis, or acrylamide gel electrophoresis is preferable, and SDS-polyacrylamide gel electrophoresis or acrylamide gel electrophoresis is more preferable, and acrylamide gel electrophoresis is particularly preferable.

It should be noted that the gel concentration (w/v) in gel electrophoresis is usually 5% to 15%, preferably 5% to 10%, and more preferably 7% to 10%. For example, in the case where acrylamide gel electrophoresis is used, the electrophoresis may be carried out with usually 5% to 15% (w/v) acrylamide, preferably 5% to 10% (w/v) acrylamide, and more preferably 7% to 10% (w/v) acrylamide. Measurement conditions of the electrophoresis and a method of detecting the target substance may be appropriately set according to a method known per se.

As a method of detecting a target nucleic acid according to the present invention after the electrophoresis, for example, a method of detecting a target nucleic acid using an intercalator can be mentioned, and this method is particularly preferable. It should be noted that, in the case of detection using an intercalator, the target nucleic acid may be detected by UV irradiation, if necessary. Specific examples of the intercalator include intercalators of the following (1) to (5) and intercalator analogues of the following (6) and (7).
(1) acridine dyes such as acridine orange,
(2) for example, ethidium compounds such as ethidium bromide, ethidium homodimer 1 (EthD-1), ethidium homodimer 2 (EthD-2), ethidium bromide monoazide (EMA), and dihydroethidium,
(3) for example, iodine compounds such as propidium iodide and hexidium iodide; for example, 7-aminoactinomycin D (7-AAD), for example, cyanine dimer dyes such as POPO-1, BOBO-1, YOYO-1, TOTO-1, JOJO-1, POPO-3, LOLO-1, BOBO-3, YOYO-3, and TOTO-3 (all of which are trade names of Molecular Probe Inc.),
(4) for example, cyanine monomer dyes such as PO-PRO-1, BO-PRO-1, YO-PRO-1, TO-PRO-1, JO-PRO-1, PO-PRO-3, LO-PRO-1, BO-PRO-3, YO-PRO-3, TO-PRO-3, and TO-PRO-5 (all of which are trade names of Molecular Probes Inc.),
(5) for example, SYTOX dyes such as SYBR Gold, SYBR Green I and SYBR Green II, SYTOX Green, SYTOX Blue, and SYTOX Orange (all of which are trade names of Molecular Probes Inc.), for example, GelRed dyes such as GelRed (trade name of Wako Pure Chemical Industries, Ltd.),
(6) compounds that binds to a minor groove of DNA double helix [for example, 4',6-diamidino-2-phenylindole (DAPI: trade name of Molecular Probes, Inc.) or the like], and
(7) compounds that specifically binds to an adenine-thymine (A-T) sequence [for example, pentahydrate (bis-benzimide) (Hoechst 33258: trade name of Molecular Probes, Inc.), trihydrochloride (Hoechst 33342: trade name of Molecular Probes, Inc.), bisbenzimide dye (Hoechst 34580: trade name of Molecular Probes, Inc.) or the like, for example, acridine dyes such as 9-amino-6-chloro-2-methoxyacridine (ACMA), and bis-(6-chloro-2-methoxy-9-acridinyl)spermine (acridine homodimer), for example, hydroxystilbamidine] .

Among the specific examples, (5) SYTOX dyes such as SYBR Gold, SYBR Green I and SYBR Green II, SYTOX Green, SYTOX Blue, and SYTOX Orange (all of which are trade names of Molecular Probes, Inc.), and GelRed dyes such as GelRed (trade name of Wako Pure Chemical Industries, Ltd.) are preferable, and GelRed dyes are more preferable.

As for the method of detecting the target nucleic acid according to the present invention, in the case where the primer is a labeled primer, the target nucleic acid may be detected according to the labeling method. Specifically, the target nucleic acid according to the present invention may be amplified in step 2 using a primer labeled with the labeling substance, and the obtained amplified product may be detected according to the labeling substance after the electrophoresis. For example, in the case where the labeling substance is a fluorescent substance, its fluorescence may be detected.

In the method using a labeled primer, it is preferable to remove the free labeled primer after amplifying the target nucleic acid according to the present invention in step 2. The method of removing the free labeled primer may be, for example, a method of precipitating the target nucleic acid according to the present invention obtained in the amplification reaction by a conventional method of precipitating a nucleic acid (an ethanol precipitation method, a precipitation method using isopropanol, or the like), and then removing the supernatant containing a free labeled primer that has not precipitated. In addition, a method of separating a primer extension product and a free labeled primer by subjecting the amplified target nucleic acid according to the present invention to gel chromatography under appropriate conditions, a separating method by electrophoresis, or the like can be mentioned.

The determination as to whether or not the target cell (the cell from which the nucleic acid in step 1 is derived) is a cancer cell is made on the basis of the results of the electrophoresis. Specifically, an electrophoretic pattern (electrophoretic migration distance or electrophoretic migration time) obtained by using a cell which has been determined to be a cancer cell or a non-cancer cell (in the present specification, the non-cancer cell contains all cells that are found to be not cancer cells) in the method of the present invention and subjecting the cell to electrophoresis, and an electrophoretic pattern (electrophoretic migration distance or electrophoretic migration time) obtained by using the target cell in the method of the present invention and subjecting the cell to electrophoresis are compared with each other, and it is determined whether the target cell is a cancer cell or a non-cancer cell on the basis of whether those patterns are the same or different.

More specifically, for example, in the case where the electrophoretic pattern (band pattern or peak pattern) obtained by using a cancer cell in the method of the present invention and then subjecting the cell to electrophoresis is the same as the electrophoretic pattern (band pattern or peak pattern) obtained by using a target cell in the method of the present invention and then subjecting the cell to electrophoresis, it is determined that the target cell is a cancer cell, and in the case where the electrophoretic patterns (band patterns or peak patterns) of both are different, it is determined that the target cell is not a cancer cell.

In addition, for example, in the case where the electrophoretic pattern (band pattern or peak pattern) obtained by using a non-cancer cell in the method of the present invention and then subjecting the cell to electrophoresis is the same as the electrophoretic pattern (band pattern or peak pattern) obtained by using a target cell in the method of the present invention and then subjecting the cell to electrophoresis, it is determined that the target cell is not a cancer cell, and in the case where the electrophoretic patterns (band patterns or peak patterns) of both are different, it is determined that the target cell is a cancer cell.

It should be noted that, regarding the comparison of the electrophoretic patterns, the entire electrophoretic profile (all bands or all peaks) obtained by electrophoresis may be compared, or the portions where the difference between a cancer cell and a non-cancer cell can be confirmed may be compared. The portion where the difference between a cancer cell and a non-cancer cell can be confirmed may be, for example, a portion where a different band or peak appears in a cancer cell and a non-cancer cell, or a portion where a band or a peak disappears in one of a cancer cell and a non-cancer cell. Specific examples thereof include a portion near the distance or time at which an amplified product of 250 bp is electrophoresed, and a portion up to near the distance or time at which an amplified product of 250 bp is electrophoresed from near the distance or time at which an amplified product of 220 bp is electrophoresed.

In addition, the determination as to whether it is a cancer cell or a non-cancer cell may be made in such a manner that the electrophoretic patterns (band patterns or peak patterns) between the cancer cell and the non-cancer cell are compared and the electrophoretic migration distance of the band or peak, the electrophoretic migration time, or the molecular weight converted therefrom, appearing or/and disappearing peculiarly in the cancer cell, is confirmed and set from the results in advance, and in the results obtained by using the target cell in the method of the present invention, the presence or absence of the band or peak corresponding to the confirmed and set electrophoretic migration distance, electrophoretic migration time, or molecular weight is confirmed (confirmation of appearance or/and disappearance of band or peak) to determine whether it is a cancer cell or a non-cancer cell. Specifically, for example, an amplified product of 220 bp not recognized in the non-cancer cell, appearance of the band or peak corresponding to the distance or time at which the amplified product is electrophoresed in the cancer cell, an amplified product of 250 bp recognized in the non-cancer cell, and disappearance of the band or peak corresponding to the distance or time at which the amplified product is electrophoresed in the cancer cell may be confirmed to determine whether it is a cancer cell or a non-cancer cell.

It should be noted that the cell from which the nucleic acid in step 1 is derived does not consist of only a single cancer cell but may be a cell group containing both a cancer cell and a non-cancer cell. In such a case, the obtained electrophoretic pattern is an electrophoretic pattern in which the electrophoretic pattern obtained from cancer cells and the electrophoretic pattern obtained from non-cancer cells are superimposed. In this case, comparison of the electrophoretic patterns (band patterns or peak patterns) is carried out, and in the case where a band or peak appearing peculiarly in cancer cells is confirmed, it is determined to be a cancer cell. Therefore, in the method of the present invention, even in the case where a band or peak appearing peculiarly in non-cancer cells is confirmed, it is determined to be a cancer cell in the case where a band or peak appearing peculiarly in cancer cells is confirmed.

A specific method of step 3 is, for example, as follows. That is, after adding electrophoresis buffer if necessary, the target nucleic acid according to the present invention amplified in step 2, together with a molecular weight marker, is subjected to electrophoresis with 5 to 15 (W/V)%, preferably 5 to 10 (W/V)%, and more preferably 7 to 10 (W/V)% acrylamide gel. Then, after staining with a GelRed dye, UV irradiation is carried out to detect a target substance. On the other hand, normal cells and cancer cells are respectively subjected to steps 1 and 2 according to the present invention to amplify target nucleic acids according to the present invention. Each nucleic acid is subjected to the electrophoresis with 5 to 15% (W/V), preferably 5 to 10 (W/V)%, and more preferably 7 to 10 (W/V)% acrylamide gel to detect a target substance in the same manner, and then the resulting band pattern and the band pattern of the target nucleic acid according to the present invention are compared to determine whether or not the target cell is a cancer cell.

It should be noted that the data (results of electrophoresis of the amplified product or/and results of determination as to whether or not it is a cancer cell) obtained from the results of step 3 can be used as data for determining whether or not an animal having a cell from which the nucleic acid in step 1 is derived (or a sample containing the cell) is affected by cancer or a cancer cell is present in the animal, and therefore the method of the present invention is also useful as a method of acquiring data to carry out such a determination.

### Primer set of present invention

The primer set of the present invention may be, for example, a combination of a forward primer consisting of the base sequence represented by SEQ ID NO: 6 and a reverse primer consisting of a contiguous base sequence containing part or all of SEQ ID NO: 8 and containing at least the base sequence represented by SEQ ID NO: 7, or a combination of a forward primer consisting of the base sequence represented by SEQ ID NO: 6 and a reverse primer consisting of the base sequence represented by SEQ ID NO: 9. Preferred is a combination of a forward primer consisting of the base sequence represented by SEQ ID NO: 6 and a reverse primer consisting of a contiguous base sequence containing part or all of SEQ ID NO: 8 and containing at least the base sequence represented by SEQ ID NO: 7, among which more preferred is a combination in which the reverse primer is a primer consisting of the base sequence represented by SEQ ID NO: 7.

### Cell cancer determination reagent of present invention

The cell cancer determination reagent of the present invention contains the primer set of the present invention, in which specific and preferred examples of the primer set are as described above. The reagent may contain a component such as a buffer solution, a stabilizer, or a preservative commonly used in the art, which does not inhibit the stability of the reagent and does not inhibit the PCR reaction. The concentration and content thereof may be appropriately set from the range commonly used in the art.

Specific examples of the buffer solution include all buffer solutions used for performing ordinary PCR or hybridization reactions, such as a Tris buffer solution, a phosphate buffer solution, a veronal buffer solution, a borate buffer solution, and a Good's buffer solution. The pH of the buffer solution is also not particularly limited, but it is usually preferably in the range of 5 to 9.

In addition, the reagent may contain a nucleic acid synthesizing enzyme (DNA polymerase, RNA polymerase, reverse transcriptase, or the like), a substrate (dNTP, rNTP, or the like) corresponding to the enzyme, a double-stranded intercalator (ethidium bromide, SYBRTM Green, or the like), a labeling detection substance such as FAM or TAMRA, or the like, if necessary.

It should be noted that the reagent of the present invention may be combined with a bisulfite reaction reagent known per se to be a reagent kit. Examples of the bisulfite reagent include those containing the compounds represented by the general formulae [1] to [8], sulfite such as sodium hydrogen sulfite or ammonium sulfite, a buffer solution, sodium hydroxide, or the like, the concentration of which in the reagent may be appropriately set so as to have the concentration described in the bisulfite reaction in step 1.

Hereinafter, the present invention will be described in more detail by way of Examples and the like, but the present invention is not limited by these Examples and the like.

### EXAMPLES

### Example 1: Analysis of FOXB2 gene (promoter region) of normal cells and cancer cells

### (1) Extraction of genomic DNA

Using the following two types of cells, genomic DNA was extracted using a QuickGene SP kit DNA tissue (manufactured by Kurashiki Boseki Co., Ltd.) according to the instructions attached.
- Human induced pluripotent stem cell (hiPS, Cell name: 201 B 7): normal cell
- Human glioma cell (U251-MG-P1): cancer cell

### (2) Bisulfite reaction

Distilled water was added to 400 ng of each genomic DNA obtained in the section (1) to prepare 10 µL of an aqueous solution. Thereafter, each solution was subjected to a bisulfite reaction using an EpiSight Bisulfite Conversion Kit (manufactured by Wako Pure Chemical Industries, Ltd.) according to the instructions attached.

### (3) Amplification of promoter region of FOXB2 gene (PCR reaction)

To 1 µL of each bisulfite reaction product obtained in the section (2), 17.3 µL of distilled water, 2.5 µL of 10×Buffer for Blend Taq (manufactured by Toyobo Co., Ltd.), 2 µL of 2 mM dNTPs (manufactured by Toyobo Co., Ltd.), 0.2 µL (0.5 U) of Blend Taq-Plus (manufactured by Toyobo Co., Ltd.), 1 µL of a forward primer (10 µM), and 1 µL of a reverse primer (10 µM) were added and mixed to prepare a reaction solution. The base sequences of these primers are as follows. In addition, the amplified chain length by these is 212 bp.
Forward primer: GTAGTTAGTTTTAGTATTTAGTTTTTGG (SEQ ID NO: 6)
Reverse primer: CCCTCTCTACTTCTCTCTCCTACCTTCTC (SEQ ID NO: 7)

Each of the reaction solutions was set in a thermal cycler (manufactured by Bio-Rad Laboratories, Inc.), and the reaction was carried out under the following reaction conditions.

### * Reaction conditions

Reaction at 94°C for 2 minutes
↓
Reaction of 36 cycles, each cycle including reaction at 94°C for 20 seconds → 55°C for 20 seconds → 72°C for 30 seconds
↓
Reaction at 72°C for 2 minutes

The amplified product obtained by the PCR reaction is SEQ ID NO: 3 below.

gcagccagct ccagcactca gtctttggcc acccggggga agacgcagag aaggcggcgg
taaacctggt gcactccgcc cgcgactgtg cgcgccagtc ggcaaccgtc ggggccagaa
gttgccagct tccgagagct gagtaggccc gaaaggccaa ggtcggagac accaggcaat
tcggagaagg caggagagag aagcagagag gg (SEQ ID NO: 3)

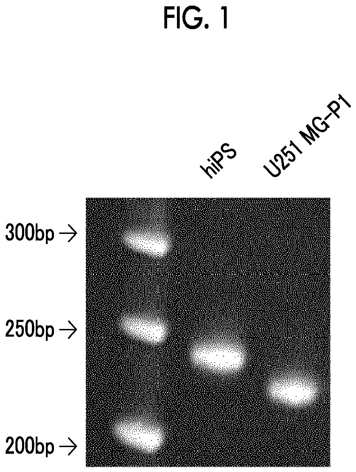

Next, 5 µL of 6×Loading Buffer Double Dye (manufactured by Nippon Gene Co., Ltd.) was added to and mixed with 25 µL of each of the obtained two PCR amplified products and 25 bp DNA Step Ladder (manufactured by Wako Pure Chemical Industries, Ltd.). The mixture was subjected to electrophoresis with SuperSep Ace, 7.5% (acrylamide gel, manufactured by Wako Pure Chemical Industries, Ltd.) at a constant current of 20 mA. After electrophoresis, the gel was stained with a GelRed nucleic acid gel staining solution (manufactured by Wako Pure Chemical Industries, Ltd.), and PCR amplified products were confirmed with a UV irradiation apparatus. The electrophoresis results of PCR amplified products obtained from normal cells of hiPS and cancer cells of human glioma cells (U251-MG-P1) are shown in Fig. 1.

From the results shown in Fig. 1, with respect to the promoter region of the FOXB2 gene, by confirming the PCR amplified product after the bisulfite reaction by acrylamide gel electrophoresis, it was found that the band in a methylated state (cancer cell) appeared on the lower molecular weight side than the band in an unmethylated state (normal cell). In other words, it was found that cancer can be determined by this method.

### Experimental Example 1: Confirmation of methylation state of genomic DNA used in Example 1

### (1) Cloning of PCR amplified product

Each of the two PCR amplified products obtained in the section (3) of Example 1 was subjected to electrophoresis on a 1.5% agarose gel. After electrophoresis, the gel was stained with a GelRed nucleic acid gel staining solution (manufactured by Wako Pure Chemical Industries, Ltd.), and PCR amplified products were recovered by a QIAquick Gel Extraction Kit (manufactured by Qiagen GmbH) according to the instructions attached.

### (2) PCR reaction for adding restriction enzyme recognition site to PCR amplified product

To 1 µL of each PCR amplified product obtained in the section (1), 17.3 µL of distilled water, 2.5 µL of 10×Buffer for Blend Taq (manufactured by Toyobo Co., Ltd.), 2 µL of 2 mM dNTPs (manufactured by Toyobo Co., Ltd.), 0.2 µL (0.5 U) of Blend Taq-Plus (manufactured by Toyobo Co., Ltd.), 1 µL of a forward primer (10 µM), and 1 µL of a reverse primer (10 µM) were added and mixed. As the forward primer, the following sequence (SEQ ID NO: 10) in which a restriction enzyme recognition site (HindIII: underlined in the sequence) is added to the forward primer of SEQ ID NO: 6 was used, and as the reverse primer, the following sequence (SEQ ID NO: 11) in which a restriction enzyme recognition site (BamHI: underlined in the sequence) is added to the reverse primer of SEQ ID NO: 7 was used.
Forward primer: TTACCATAAGCTT GTAGTTAGTTTTAGTATTTAGTTTTTGG (SEQ ID NO: 10)
Reverse primer: TAATTAAGGATCC CCCTCTCTACTTCTCTCTCCTACCTTCTC (SEQ ID NO: 11)

Each of the reaction solutions was set in a thermal cycler and the reaction was carried out under the following conditions.

### * Reaction conditions

Reaction at 94°C for 2 minutes
↓
Reaction of 10 cycles, each cycle including reaction at 94°C for 20 seconds → 55°C for 20 seconds → 72°C for 30 seconds
↓
Reaction at 72°C for 2 minutes

Next, 5 µL of 6×Loading Buffer Double Dye (manufactured by Nippon Gene Co., Ltd.) was added to and mixed with 25 µL of each PCR amplified product, and the mixture was subjected to electrophoresis on a 1.5% agarose gel. After electrophoresis, the gel was stained with a GelRed nucleic acid gel staining solution (manufactured by Wako Pure Chemical Industries, Ltd.), and PCR amplified products were recovered by a QIAquick Gel Extraction Kit (manufactured by Qiagen GmbH) according to the instructions attached.

### (3) Restriction enzyme treatment

To 43 µL of each PCR amplified product obtained in the section (2), 5 µL of 10×B Buffer (manufactured by Nippon Gene Co., Ltd.), 1 µL of HindIII (manufactured by Nippon Gene Co., Ltd.) and 1 µL of BamHI (manufactured by Nippon Gene Co., Ltd.) were added and they are mixed and the mixture was incubated at 37°C for 1 hour. Similarly, distilled water was added to 500 ng of pUC19 (manufactured by Nippon Gene Co., Ltd.) to make 43 µL, and further 1 µL of HindIII (manufactured by Nippon Gene Co., Ltd.) and 1 µL of BamHI (manufactured by Nippon Gene Co., Ltd.) were added and mixed, and the mixture was incubated at 37°C for 1 hour. Subsequently, 250 µL of binding buffer (5.5 M guanidine hydrochloride (manufactured by Wako Pure Chemical Industries, Ltd.) and 20 mM Tris-HCl pH 6.6 (manufactured by Wako Pure Chemical Industries, Ltd.)) was added and mixed, and then the mixture was transferred to an ECONOSPIN (manufactured by Gene Design, Inc.) and centrifuged at 12000×g and room temperature for 1 minute to remove the solution in the tube. Thereafter, 500 µL of washing buffer [2 mM Tris-HCl, pH 7.5 containing ethanol at 80% (manufactured by Nippon Gene Co., Ltd.)] was added and the mixture was centrifuged at 12000×g and room temperature for 1 minute to remove the solution in the tube. After further centrifugation at 12000×g and room temperature for 1 minute, the tube was replaced with a fresh tube and 25 µL of elution buffer (10 mM Tris-HCl, pH 8.5) (manufactured by Nippon Gene Co., Ltd.) was added, and the mixture was centrifuged at 12000×g and room temperature for 1 minute to remove the solution. As a result, the restriction enzyme-treated PCR amplified product and pUC19 were recovered.

### (4) Transformation

To 1 µL of each restriction enzyme-treated PCR amplified product, 1 µL of restriction enzyme-treated pUC19 and 2 µL of a DNA Ligation Kit (Mighty Mix) (manufactured by Takara Bio Inc.) were added and mixed, and the mixture was incubated at 16°C for 1 hour. Next, a total volume of 4 µL was transfected into ECOS™ Competent *E. coli* DH5α (manufactured by Nippon Gene Co., Ltd.) which was then spread on an LB agar medium supplemented with ampicillin. After that, the colonies were cultured in an LB medium supplemented with ampicillin at 37°C for 16 hours. In addition, extraction of a plasmid was carried out using a Plasmid Kit SII (manufactured by Kurabo Industries Ltd.) according to the instructions attached.

Using the resulting plasmid, the base sequence was analyzed using contract sequencing service by Takara Bio Inc.

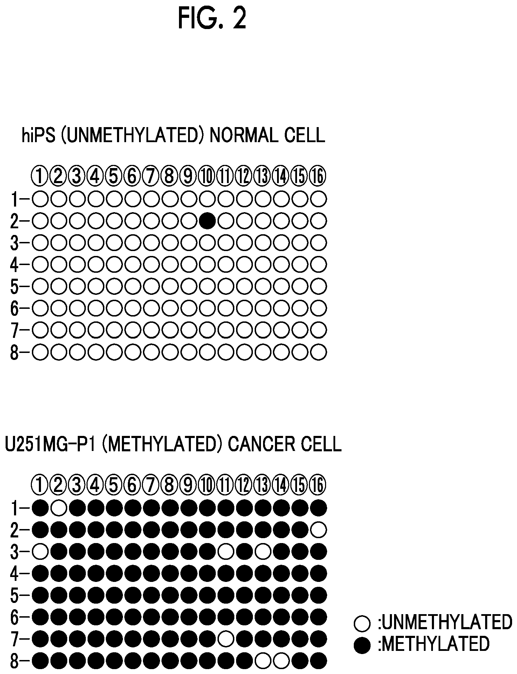

On the basis of the results of base sequencing, a diagram showing whether cytosine of a CpG dinucleotide is unmethylated cytosine or methylated cytosine is shown in Fig. 2. It should be noted that ○ in the diagram indicates that cytosine of a CpG dinucleotide is unmethylated, and ● indicates that cytosine of a CpG dinucleotide is methylated.

From the results in Fig. 2, it was confirmed that cytosine in normal cells of hiPS was almost unmethylated cytosine, and cytosine in cancer cells of human glioma cells (U251-MG-P1) was mostly methylated. Therefore, according to the method of the present invention, it was shown that methylation (methylated cytosine) or unmethylation (unmethylated cytosine) of cytosine in the promoter region of the FOXB2 gene in cells can be distinguished by a simple method. That is, according to the method of the present invention, it was found that whether or not a cell is a cancer cell can be distinguished by a simple method.

### Example 2: Analysis of FOXB2 gene (promoter region) using cloned plasmid

PCR amplification was carried out using plasmids (PCR amplified chain length: 212 bp) of the promoter region of the FOXB2 gene derived from hiPS and U251-MG-P1 obtained in Experimental Example 1 as templates, respectively, and then the PCR amplified products were confirmed by acrylamide gel electrophoresis.

Specifically, to 1 µL (50 ng) of each plasmid DNA, 17.3 µL of distilled water, 2.5 µL of 10×Buffer for Blend Taq (manufactured by Toyobo Co., Ltd.), 2 µL of 2 mM dNTPs (manufactured by Toyobo Co., Ltd.), 0.2 µL (0.5U) of Blend Taq-Plus (manufactured by Toyobo Co., Ltd.), 1 µL of a forward primer (10 µM) and 1 µL of a reverse primer (10 µM) were added and mixed to obtain a reaction solution. It should be noted that the same forward primer and reverse primer as those described in the section (3) of Example 1 were used.

Each of the reaction solutions was set in a thermal cycler and the reaction was carried out under the following conditions.

### * Reaction conditions

Reaction at 94°C for 2 minutes
↓
Reaction of 10 cycles, each cycle including reaction at 94°C for 20 seconds → 55°C for 20 seconds → 72°C for 30 seconds
↓
Reaction at 72°C for 2 minutes

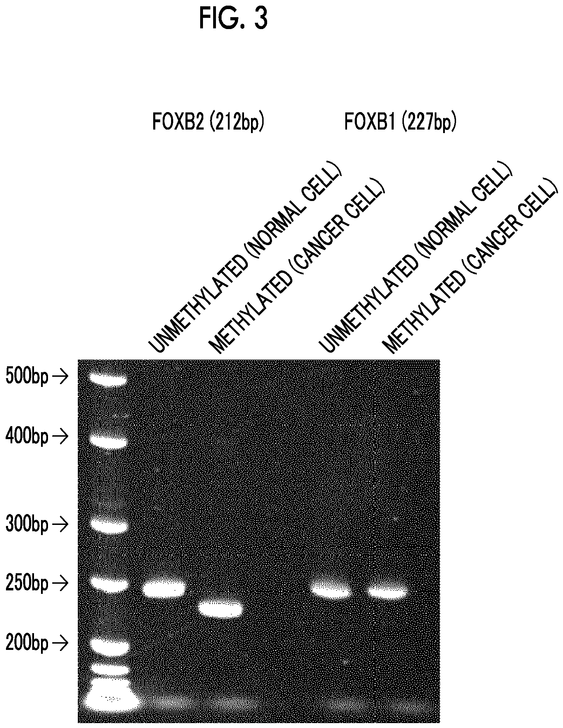

Subsequently, 5 µL of 6×Loading Buffer Double Dye (manufactured by Nippon Gene Co., Ltd.) was added to and mixed with each of 25 µL of the PCR amplified product and 25 bp DNA Step Ladder (manufactured by Wako Pure Chemical Industries, Ltd.). The mixture was subjected to electrophoresis with SuperSep Ace, 7.5% (acrylamide gel, manufactured by Wako Pure Chemical Industries, Ltd.). After electrophoresis, the gel was stained with a GelRed nucleic acid gel staining solution (manufactured by Wako Pure Chemical Industries, Ltd.), and PCR amplified products were confirmed with a UV irradiation apparatus. The results are shown in Fig. 3.

### Comparative Example 1: Analysis of FOXB1 gene (promoter region) of normal cells and cancer cells

Using a human induced pluripotent stem cell (hiPS) and a human glioma cell (U251-MG-P1), the PCR amplified product of the promoter region of the FOXB1 gene was electrophoresed in the same manner as in Example 1, except that a forward primer as set forth in SEQ ID NO: 12 below and a reverse primer as set forth in SEQ ID NO: 13 below were used in place of the forward primer and the reverse primer in the section (3) of Example 1. The results are shown in Fig. 3 together with the results of FOXB2 of Example 2.
Forward primer: GTAGAGGAAATAGAATATTTTAGTTAAG (SEQ ID NO: 12)
Reverse primer: CTTAACCAATAAAAATCACTTCCAACTC (SEQ ID NO: 13)

It should be noted that the electrophoresed amplified product is a FOXB1 gene of SEQ ID NO: 14 having an amplified chain length of 227 bp. The base sequence has been described as bases 92714 to 92940 in Genbank Accession No. AC009654.

GCAGAGGAAA CAGAATACCC CAGCCAAGCA ATTTCCATGT
CAAACATCAT CCGCGCGGCT
GCTCCATCTG TGAACGTGAG TGGTCGCCTG GCTCCCTCTT
CCGCGGCGGC CGCTTCCTCA
TACCTTCACA CGGCGCACAC CGGCCGGCGC GTCCAGCTGC

CTGCCCAGTG GCCGAGGCTC
TTCCCCCTCG CCCAGTCTTG AGCTGGAAGT GATTCCTATT GGCCAAG
(SEQ ID NO: 14)

From the results in Fig. 3, with respect to the promoter region of the FOXB2 gene, it was also found that the presence or absence of methylated cytosine can be similarly distinguished by acrylamide gel electrophoresis of a PCR amplified product using a plasmid after being subjected to the bisulfite reaction. On the other hand, with respect to the promoter region of the FOXB1 gene, it was found that the presence or absence of methylated cytosine cannot be distinguished since there is no difference in electrophoretic migration distance by the presence/absence of methylated cytosine.

### Comparative Example 2: Analysis of various FOXB2 genes

For the following regions (amplified sequences 1 to 3) different from the base sequence amplified in Example 1, the FOXB2 gene was amplified and the amplified product was subjected to electrophoresis. Amplified sequence 1 is a base sequence of a part of the promoter region of the FOXB2 gene and a part of the structural gene of the FOXB2 gene, and amplified sequence 2 and amplified sequence 3 are a part of the base sequence represented by SEQ ID NO:3 which is the promoter region of the FOXB2 gene.
Amplified sequence 1: SEQ ID NO: 24 
GGAATTAGTG GGGGCAGCCA GGCCCCAGGA ACGGAGTGCG
GAGAGATTCT GTGGTCCAGT GCGGGCCGGA GGGCGGTGGA GGAGCCGGGG
GCGATGCCGC GGCCGGGGAA GAGCTCGTAC AGCGACCAAA AACCGCCCTA
CTCTTACATC TCGCTGACCG CCATGGCAAT CCAGCACTCG GCCGAGAAGA
TGCTGCCGCT GAGCGACATC TACAAGTTCA TCATGGAGCG CTTCCCCTAC
TACCGCGAGC ACACACAGCG CTGGCAGAAC AGCCTGCGCC ACAACCTCTC
CTTCAACGAC TGCTTCATCA AGATTCCGCG GAGGCCCGAC CAGCCTGGCA
AGGGTAGCTT CTGGG
Amplified sequence 2: SEQ ID NO: 25 
GGTAAACCTG GTGCACTCCG CCCGCGACTG TGCGCGCCAG
TCGGCAACCG TCGGGGCCAG AAGTTGCCAG CTTCCG
Amplified sequence 3: SEQ ID NO: 26

GTTGCCAGCT TCCGAGAGCT GAGTAGGCCC GAAAGGCCAA

GGTCGGAGAC ACCAGGCAAT TCGGAGAAGG CAGGAGAGAG AAGCAGAGAG GG

Specifically, experiments were carried out in the same manner as in Example 1, except that forward primer 1 and reverse primer 1 shown below were used in place of the forward primer and the reverse primer in the section (3) of Example 1, whereby the PCR amplified product (amplified sequence 1) of the FOXB2 gene was electrophoresed using a human induced pluripotent stem cell (hiPS) and a human glioma cell (U251-MG-P1). In addition, experiments were carried out in the same manner as in Example 1, except that, in the case of hiPS, unmethylated forward primer 2 and unmethylated reverse primer 2 shown below were used in place of the forward primer and the reverse primer, and in the case of U251-MG-P1, methylated forward primer 2 and methylated reverse primer 2 shown below were used in place of the forward primer and the reverse primer, whereby the PCR amplified product (amplified sequence 2) of the promoter region of the FOXB2 gene was electrophoresed. In addition, experiments were carried out in the same manner as in Example 1, except that, in the case of hiPS, unmethylated forward primer 3 and reverse primer 3 shown below were used in place of the forward primer and the reverse primer, and in the case of U251-MG-P1, methylated forward primer 3 and reverse primer 3 shown below were used in place of the forward primer and the reverse primer, whereby the PCR amplified product (amplified sequence 3) of the promoter region of the FOXB2 gene was electrophoresed.

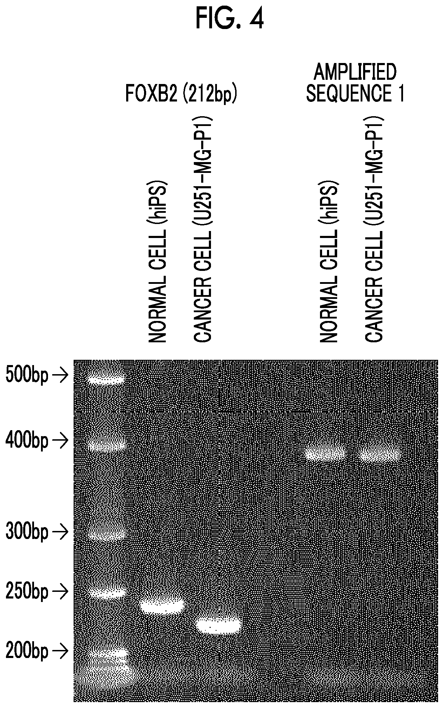

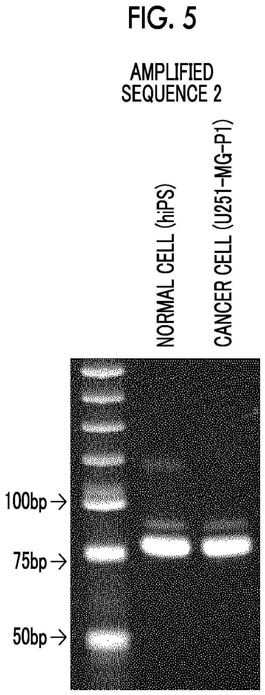

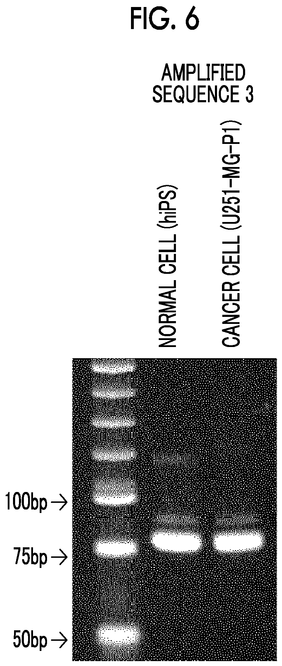

The electrophoresis results of amplified sequence 1 are shown in Fig. 4. It should be noted that, on the left side of Fig. 4, the results of carrying out Example 2 again are shown as a comparison. In addition, the electrophoresis results of amplified sequence 2 are shown in Fig. 5, and the electrophoresis results of amplified sequence 3 are shown in Fig. 6. It should be noted that the amplified chain lengths of the electrophoresed amplified products are 355 bp (amplified sequence 1), 76 bp (amplified sequence 2), and 92 bp (amplified sequence 3) in order, respectively.

Forward primer 1: GGAATTAGTGGG GGTAGTTAGGTTTTAGG (SEQ ID NO: 15)
Reverse primer 1: CCCAAAAACTACCCTTACCAAACTAATC (SEQ ID NO: 16)

Unmethylated forward primer 2: GGTAAATTTGGTGTATTTTGTTTG (SEQ ID NO: 17)
Methylated forward primer 2: GGTAAATTTGGTGTATTTCGTTCG (SEQ ID NO: 18)
Unmethylated reverse primer 2: CAAAAACTAACAACTTCTAACCCC (SEQ ID NO: 19)
Methylated reverse primer 2: CGAAAACTAACAACTTCTAACCCC (SEQ ID NO: 20)

Unmethylated forward primer 3: GTTGTTAGTTTTTGAGAGTTGAGTAG (SEQ ID NO: 21)
Methylated forward primer 3: GTTGTTAGTTTTCGAGAGTTGAGTAG (SEQ ID NO: 22)
Reverse primer 3: CCCTCTCTACTTCTCTCTCCTACCTTCTC (SEQ ID NO: 23)

From the results in Figs. 4 to 6, it was found that the sequence in which the presence or absence of methylated cytosine can be distinguished by electrophoresis according to the method of the present invention is only a specific region of the promoter region of the FOXB2 gene. That is, it was found that it is necessary to have at least a sequence having the CG base in the base sequence represented by SEQ ID NO:3 confirmed in Example 1 (that is, SEQ ID NO: 2).

### Example 3: Analysis of FOXB2 gene (promoter region) using cells derived from breast cancer patients

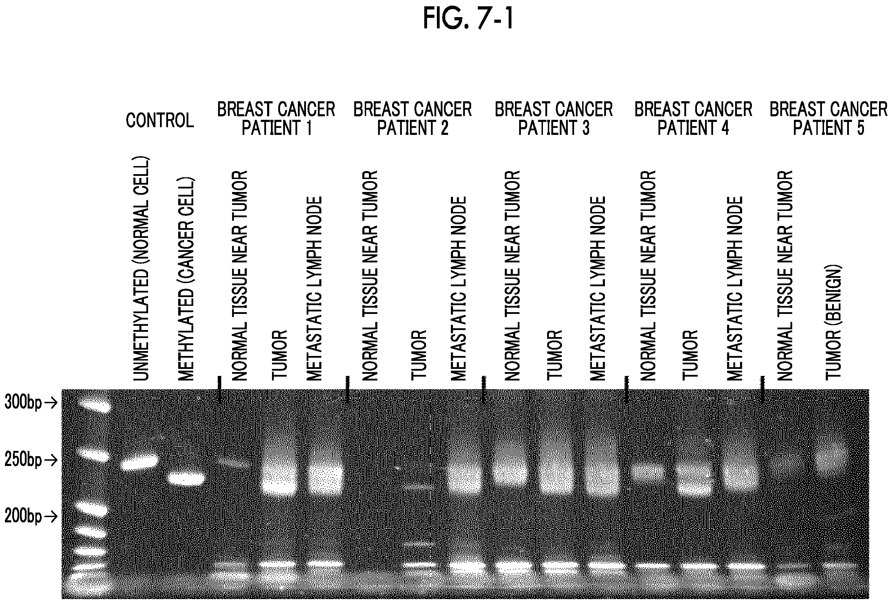

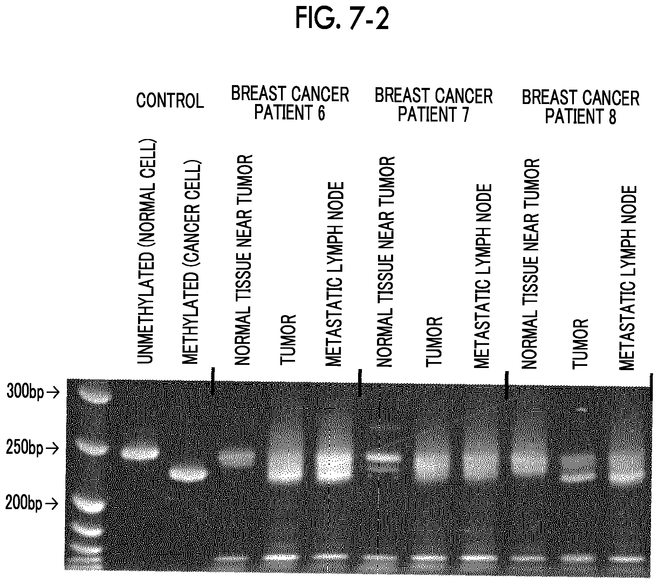

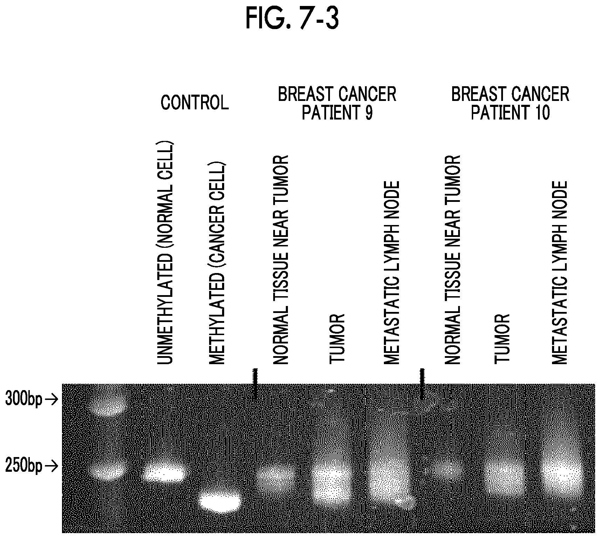

PCR amplified products of the promoter region of the FOXB2 gene were electrophoresed in the same manner as in Example 1, except that mammary gland cells of 8 breast cancer patients were used as the cells in the section (1) of Example 1. The electrophoresis results are shown in Figs. 7-1, 7-2, and 7-3, respectively. It should be noted that the cell of breast cancer patient 5 in Fig. 7-1 is a benign tumor.

From the results of Figs. 7-1 to 7-3, according to the method of the present invention, electrophoretic migration was confirmed in a tumor or metastatic lymph node at the same position as that of cancer cells, and therefore it could be determined that the tumor or metastatic lymph node is a cancer cell (the cell is cancerized). In addition, it was also found that even normal tissues near the tumor can be determined to be normal cells and that benign tumors can also be determined to be normal cells.

### Example 4: Analysis of methylation of FOXB2 gene (promoter region) using cells

### derived from colorectal cancer patients

PCR amplified products of the promoter region of the FOXB2 gene were electrophoresed in the same manner as in Example 1, except that colorectal cells of 8 colorectal cancer patients were used as the cells in the section (1) of Example 1.

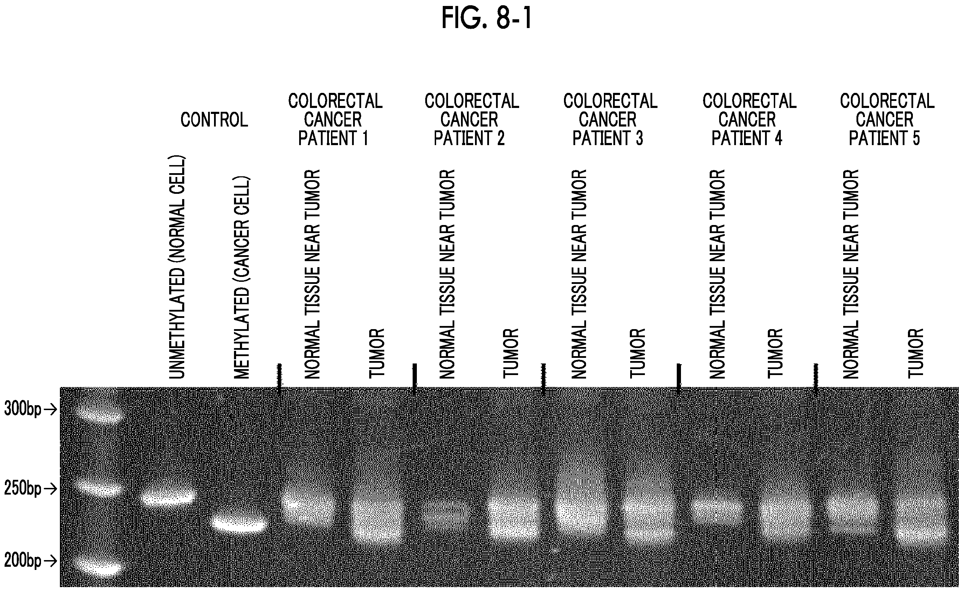

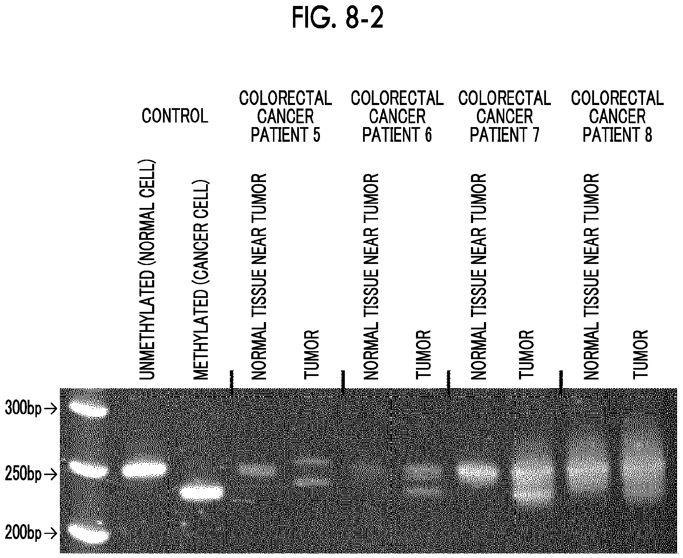

The electrophoresis results are shown in Figs. 8-1 and 8-2, respectively.

From the results of Figs. 8-1 and 8-2, according to the method of the present invention, it was found that, even in the case where cells derived from colorectal cancer are used, electrophoretic migration was confirmed in a tumor at the same position as that of cancer cells, and therefore the tumor can be determined to be a cancer cell.

The positional relationships of the sequences used in the foregoing Examples and Comparative Examples are shown below.

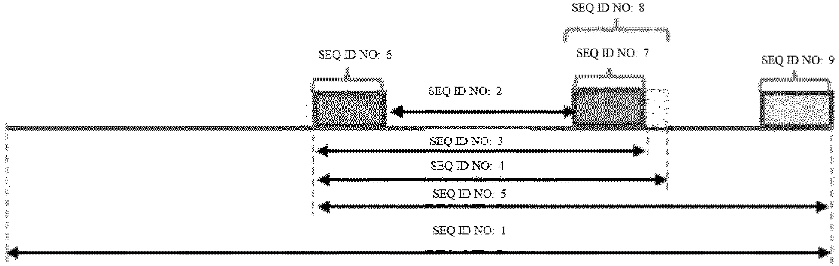
SEQ ID NO: 8
SEQ ID NO: 6
SEQ ID NO: 7
SEQ ID NO: 9
SEQ ID NO: 2
SEQ ID NO: 3
SEQ ID NO: 4
SEQ ID NO: 5
SEQ ID NO: 1

## Claims

1. A cancer cell determination method, comprising the following steps 1 to 3:
(1) step 1 of subjecting a cell-derived nucleic acid to a bisulfite reaction, the nucleic acid containing at least a base sequence represented by SEQ ID NO: 2 and containing part or all of a base sequence represented by SEQ ID NO: 1;
(2) step 2 of amplifying, among reaction products obtained in step (1), a bisulfite reaction product of a nucleic acid containing at least the base sequence represented by SEQ ID NO: 2 and consisting of part or all of the base sequence represented by SEQ ID NO: 1; and
(3) step 3 of determining whether or not the cell is a cancer cell based on the results of subjecting the amplified product obtained in the step (2) to electrophoresis.

2. The determination method according to claim 1, wherein the nucleic acid to be amplified in the step (2) is a bisulfite reaction product of a nucleic acid containing at least the base sequence represented by SEQ ID NO: 2 and consisting of part or all of a base sequence represented by SEQ ID NO: 5.

3. The determination method according to claim 1, wherein the nucleic acid to be amplified in the step (2) is a bisulfite reaction product of a nucleic acid containing at least the base sequence represented by SEQ ID NO: 2 and consistingof part or all of a base sequence represented by SEQ ID NO: 4.

4. The determination method according to claim 1, wherein the nucleic acid to be amplified in the step (2) is a bisulfite reaction product of a nucleic acid containing at least the base sequence represented by SEQ ID NO: 2 and consistingof part or all of a base sequence represented by SEQ ID NO: 3.

5. The method according to any one of claims 1 to 4, wherein the amplifying step of the step (2) is carried out by a PCR reaction.

6. The method according to claim 5, wherein the PCR reaction is carried out using a forward primer consisting of a base sequence represented by SEQ ID NO: 6; and a reverse primer consistingof a base sequence of part or all of SEQ ID NO: 8 and containing at least a base sequence represented by SEQ ID NO: 7, or a reverse primer consisting of a base sequence represented by SEQ ID NO: 9.

7. The method according to any one of claims 1 to 6, wherein the electrophoresis is gel electrophoresis.

8. A primer set, comprising:
a forward primer consisting of a base sequence represented by SEQ ID NO: 6; and
a reverse primer consisting of a base sequence containing part or all of SEQ ID NO: 8 and containing at least a base sequence represented by SEQ ID NO: 7, or a reverse primer consistingof a base sequence represented by SEQ ID NO: 9.

9. A reagent for cancer cell determination, comprising:
the primer set according to claim 8.
